# EUROPEAN PATENT SPECIFICATION

(11) **EP 4 027 916 B1**
(45) Date of publication and mention of the grant of the patent: **10.01.2024**
(21) Application number: 20829333.2
(22) Date of filing: 19.11.2020
(51) Int. Cl.: A61B 17/22, A61M 25/09

(54) **DELIVERY ASSIST WIRE-CATHETER ASSEMBLY**
EINFÜHRHILFE-DRAHT-KATHETER-BAUGRUPPE
ENSEMBLE CATHÉTER-FIL D'ASSISTANCE DE DISTRIBUTION

(43) Date of publication of application: 20.07.2022
(73) Proprietor: ForFlow, UAB, 04124 Vilnius (LT)
(72) Inventor: Sirvinskas, Audrius, 05120 Vilnius (LT); Pranulis, Vytautas, 04352 Vilnius (LT)
(74) Representative: Mooser, Sebastian Thomas
(86) International application number: PCT/IB2020/060893
(87) International publication number: WO 2022/106867

(56) References cited:
- JP-A- 2005 211 524
- US-A1- 2002 091 372
- US-A1- 2009 270 808
- US-A1- 2010 204 672
- US-A1- 2016 089 173

## Description

### TECHNICAL FIELD

The present disclosure relates generally to interventional neuro-radiology procedures and more particularly to a Delivery assist wire-catheter assembly wherein the Delivery assist wire has special variating diameter dedicated to assist and drive the delivery of neuro aspiration catheter or another neuro catheter to a thrombus area and to pull back out of aspiration catheter thereby creating blood and clot suction flow and providing aspiration effect.

### BACKGROUND

It is quite obvious to say that timing for treating patient with ischemic stroke is absolute critical in achieving good clinical outcomes, meaning that the faster the recanalization of thrombosed vessel achieved the better is clinical outcomes. Being aspiration thrombectomy has become a nearly first of the choose treatment option among interventional radiologists to treat ischemic stroke their society has gathered solid experience within this practice. As more practice is accumulated more and more physicians emphasize the importance to be able to reach thrombosed vessel area by advancing aspi catheter by easy and fast means. There are couple of solutions (products) available on the market already, but it is not good enough and it is not simple enough to enable physicians to use it on a daily basis to advance and drive aspi catheters threw neuro vessels, especially through challenging anatomies like syphon area etc. The guiding of aspi catheter tube, precise manoeuvring of the distal part of the Delivery assist wire-catheter assembly and sucking of thromboectomy clot through the catheter are the main challenges faced during thromboectomy. Therefore, a natural demand has been growing to seek for fast, simple and effective ways to steer and advance aspi catheters to the desired thrombosed area whether it's ACI distal segment, M1 or M3 segment.

US201762583613 discloses the device which is used to the remove a thrombus from the vasculature. It includes an aspiration catheter and a thrombus retrieval device that extends through the lumen of the aspiration catheter. An expandable braided assembly extends over a distal region of the retrieval device, and an activation wire extends through the lumen of the retrieval device to attach to and control the expansion of the braided assembly. Applying tension to the activation wire causes the braided assembly to expand to a diameter of the practitioner's choosing. For example, the practitioner may apply a first level of tension to the activation wire to deploy the braided assembly to a first diameter and then later change the diameter by applying a different level of tension. The expanded braided assembly contacts the thrombus and is pulled proximally toward the aspiration catheter to assist in thrombus removal.

US201916699563 provides systems for less invasive medical procedures comprise a filter device mounted on an integrated guiding structure and an aspiration catheter. These components can be used together or separately, and the system can be used with other medical devices that are designed for less invasive procedures, such as procedures in a patient's vasculature. The catheter can have a radiopaque band that is held in place under metal wire embedded within the polymer forming the tube of the catheter. In some embodiments, the aspiration catheter has a small diameter distal portion that can access into small diameter vessels in which the distal portion has a smaller average diameter than the remaining tube of the catheter.

EP3600517 provides a guide wire-catheter assembly, comprising: a catheter tube having a longitudinal channel, and a guide wire configured to be movable in the longitudinal channel characterized in that, the catheter tube comprises a bendable part near its distal end, wherein the bendable part comprises, in the circumferential direction of the catheter tube, a varying flexibility, such that exerting a longitudinal compression force in a proximal direction at a compression location distally of the distal end part results in bending of the bendable part, and in that the guide wire comprises an expandable part, wherein the expandable part is movable between a non-expanded position, in which a cross section of the expandable part is smaller than a smallest cross section of the longitudinal channel of the catheter tube and an expanded position, in which a cross section of the expandable part is larger than the smallest cross section of the longitudinal channel of the catheter tube, wherein, when the expandable part is in the non-expanded position, the guide wire can be moved completely in and out of the longitudinal channel of the catheter tube, and wherein, when the expandable part is in the non-expanded position, the expandable part is configured to exert the longitudinal compression force on the compression location in order to bend the bendable part of the catheter tube. JP 2005 211524 A discloses a guidewire for inserting a catheter whereby the guidewire has an enlarged diameter front end.

US 2009/270808 A1 discloses an aspiration catheter having an inner slidable sealing member to create a suction force and a separate eccentrically placed guidewire.

A drawback of the conventional guide wire-catheter assemblies is that advancing the guide wire through the catheter tube channel, in order to guide the catheter through a body vessel, usually results in shape change of the distal end of the guide wire-catheter assembly, which makes precise manoeuvring of the distal end of the guide wire-catheter assembly difficult.

Multiple devices have been introduced with integrated guiding structure and an aspiration catheter. However, usually it is needed to use additional devices like neuro micro-wires, microcatheters, support catheters, to guide the catheter through vascular system. There is a need for an improved delivery assist wire-device with special variating diameter which is configurated to be inserted into the catheter and which is used for assisting and driving the delivery of neuro aspiration catheter that is more effective for removing thrombus from the vascular system.

### BRIEF DESCRIPTION OF THE INVENTION

Is it known from prior art that guide wire-catheter assemblies comprise a catheter tube having a longitudinal channel and a guide wire, configured to be movably arranged in the longitudinal channel of the catheter tube. The purpose of this invention is to provide simple, however highly effective technical solution to guide and track thrombectomy aspiration catheter through vessels anatomy with easy and prompt drive to the thrombus area and afterwards pull back the Delivery assist wire out of aspiration catheter thereby creating blood and clot suction flow and providing aspiration effect. This is achieved by Delivery assist wire which has variable diameter which is thicker diameter at the distal working part where major driving and steering forces are needed to push aspiration catheter through challenging and torturous vessel anatomies (e.g. avoid snagging at side vessel "Y" part branching). The major innovation is to have thicker distal working part of the Delivery assist wire that is configured to fill into aspiration catheter distal part inner lumen and improve deliverability of catheter through torturous vessels. The Delivery assist wire and aspiration catheter lumen wall would create a kind of "uni-body" structure that being together would slide much easier through challenging vessel anatomies. The invention is defined by claim 1. Further embodiments of the invention are defined by the dependent claims. Methods are not claimed per sé.

In one embodiment the Delivery assist wire-catheter assembly comprises the delivery assist wire and the aspiration catheter wherein the Delivery assist wire of the invention is guide wire which is configured for insertion into the longitudinal channel of aspiration catheter. The Delivery assist wire has a distal tip part, two additional parts, a distal working part, and a base part.

The base part of guide wire has a smaller diameter relative to the diameter of the distal working part of guide wire, wherein base part of guide wire has diameter maximal 1 mm, preferably 0.89 mm.

The Delivery assist wire comprises:
(a) the distal tip part which has diameter maximal 1 mm, preferably 0.89 mm;
(b) the additional parts which are in the shape of distal inclining conical and proximal declining conical, negative conical or perpendicular part;
(c) the distal working part which has diameter in the interval between 0.9 mm and 1.88 mm, preferably 1.27 mm;
(d) the base body part which has diameter maximal 1 mm, preferably 0.89 mm.

The distal working part of Delivery assist wire has an outer diameter from 60 to 99 percent of the average inner diameter of the aspi catheter lumen at the distal aspi catheter end.

The structure of the Delivery assist wire is selected from spring wire or braided wire, wherein the spring wire is composed of a core wire and a spring wire mounted on the top of the core or wherein braided multiple wires form single body of Delivery assist wire. Both spring wire and braided wire has hydrophilic coating for smooth push-ability, passage, and drive.

In another embodiment mentioned Delivery assist wire is configured for insertion into the aspiration catheter wherein the catheter is femoral, brachial or radial vessel catheter.

The Delivery assist wire may be made of any suitable material, such as stainless steel, nitinol, or other suitable metal materials.

In an embodiment the Delivery assist wire is configured for insertion into the aspi catheter and wherein the aspi catheter is configured to reach the thrombus following to the path of Delivery assist wire (step-by-step).

In another embodiment the Delivery assist wire is configured for insertion into the aspi catheter and wherein the aspi catheter and the guide wire are configured for simultaneous reaching of the thrombus area (uni-body).

Further, the Delivery assist wire is configured to work as support body for the proximal and middle ends of the aspi catheter to keep on pushing the aspi catheter forward.

Delivery assist neuro-wire is configured for use in thrombectomy aspiration neuro catheter and is configured for use to steer, drive and advance forward aspiration catheter to the intracranial vessel areas from carotid up to M1-M3 vessel segments.

When the Delivery assist wire drives and delivers the aspiration catheter to the thrombus area, the Delivery assist wire by continuous movement is being pulled back out of aspiration catheter (while aspi catheter is on still hold) thereby creating a negative blood flow and thus enabling the remove of the thrombus clot out of the vessel.

By using Delivery assist wire together with the aspiration catheter to advance aspiration catheter to thrombus area there is no need to use any other additional devices like neuro micro-wires, microcatheters, support catheters or any other similar devices, except the initial long introduce sheath alone.

### BRIEF DESCRIPTION OF FIGURES

Fig. 1 shows Delivery assist wire-catheter assembly;
Fig. 2 shows the Delivery assist wire;
Fig. 3 shows a top view of an operating Delivery assist wire;
Fig. 4 shows pull back aspiration effect provided by the Delivery assist wire-catheter assembly.

### DETAILED DESCRIPTION OF THE INVENTION

The Delivery assist wire is provided in various diameter versions depending on for which inner diameter aspiration catheter it is used to go with. The base body part diameter remains constant for each model of Delivery assist wire, while the diameters of distal and distal tip parts may differ in the same wire following by the diameters listed above. Currently there are officially known and used neuro thrombectomy aspiration catheters which have inner diameter between 1.5 mm and 1.88 mm inner diameter, but in case the inner lumen diameter of aspi catheter will change (e.g. will increase) than the width of the wider distal part of Delivery assist wire will also increase to adopt to the needs of wider/narrower inner lumen of aspi catheter.

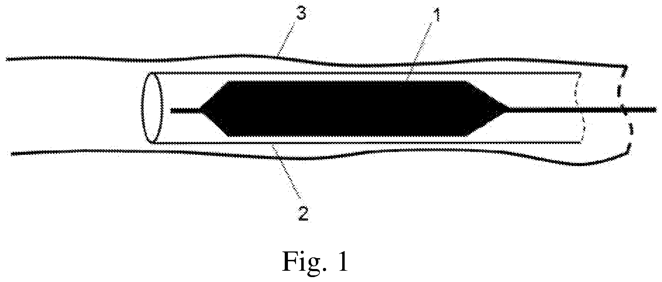

Fig. 1 shows the Delivery assist wire-catheter assembly which comprises the Delivery assist wire (1) and catheter tube (2) wherein the Delivery assist wire (1) is configured for the insertion into the catheter tube (2) with the distal tip part (4) forward.

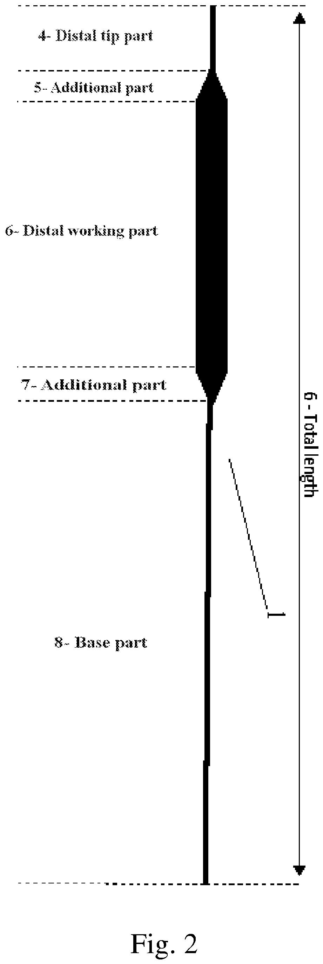

Figure 2 shows the Delivery assist wire (1) which comprises the distal tip part (4), two additional parts (5, 7), distal working part (6), and base body part (8).

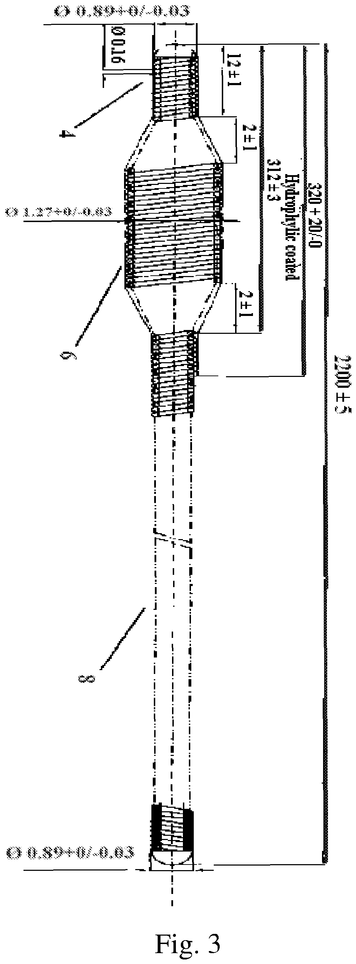

Fig. 3 shows top view of an operating Delivery assist wire (1) wherein the distal tip part (4) diameter is maximal 1 mm, preferably 0.89 mm.

The distal working part (6) together with additional parts (5,7) has any suitable length, for example, which is from 10 mm to 500 mm. The diameter of distal working part (6) is from 0.9 mm to 1.88 mm, preferably 1.27 mm.

The Delivery assist wire (1) is configured to insert into the aspiration catheter tube (2). Figure 3 shows best mode of invention in which the Delivery assist wire (1) has the distal tip part (4) which diameter is 0.89 mm and the distal working part (6) which diameter is 1.27 mm. The distal working part (6) has two additional parts (5, 7) which are in the shape of distal inclining conical and proximal declining conical, negative conical or perpendicular part - going from diameter maximal 1 mm, preferably 0.89 mm into diameter of interval between 0.9 mm and 1.88 mm, preferably 1.27 mm;

The Delivery assist wire (1) is advanced into the intracranial vessel via femoral, brachial or radial vessel introducer.

The total length of the Delivery assist wire (1) may be dependent on the application for which it is used, and may be, for example, in the range of 2.0 - 3.3 m.

The advancement of aspiration catheter can be performed by either of two methods:
1) The drive and the delivery of the aspiration catheter (2) is performed on "step-by-step" principal where the Delivery assist wire (1) is pushed forward at certain length, then pushing is stopped and while holding the Delivery assist wire (1) still the aspiration catheter (2) is pushed following to the path of Delivery assist wire as on the "mono-rail" path. The "step-by-step" movements are repeated until Delivery assist wire (1) reaches (but does not cross) the thrombus.
2) The other method is to advance aspiration catheter (2) together with the Delivery assist wire (1) as "uni-body" structure, meaning that both devices is advanced simultaneously together at the same pushing forward movement. During this method the distal tip (4) of the Delivery assist wire (1) invariably is sticking out of the aspiration catheter (2) to form the conical distal structure consisting of aspiration catheter (2) and Delivery assist wire (1) as distal "uni-body shape tip".

So, by means of any of two methods the Delivery assist wire (1) helps to advance aspiration catheter (2) to come to thrombus as close as possible. The Delivery assist wire (1) also works as a support body at more proximal parts of aspiration catheter (2) wherein such support is needed to keep on pushing the aspiration catheter (2) forward and having challenges at more middle part of the catheter area where additional inner support is needed.

Once the aspiration catheter (2) gets close enough to thrombus, then Delivery assist wire (1) is pulled back into the aspiration catheter (2) so that there is no distal tip (4) of the Delivery assist wire (1) sticking out of the aspiration catheter (2), just the distal tip of the aspiration catheter (2) alone is pushed forward to touch the thrombus. Once "touching" is achieved the Delivery assist wire (1) is totally removed from aspiration catheter.

The Delivery assist wire (1) is intended to assist the delivery of aspiration catheter or other neuro catheter (e.g. distal access catheter) to vessel segments. The Delivery assist wire (1) is configured for use in thrombectomy aspiration neuro catheter and to steer, drive and advance forward aspiration catheter (2) to the intracranial vessel areas from carotid up to M1-M3 vessel segments.

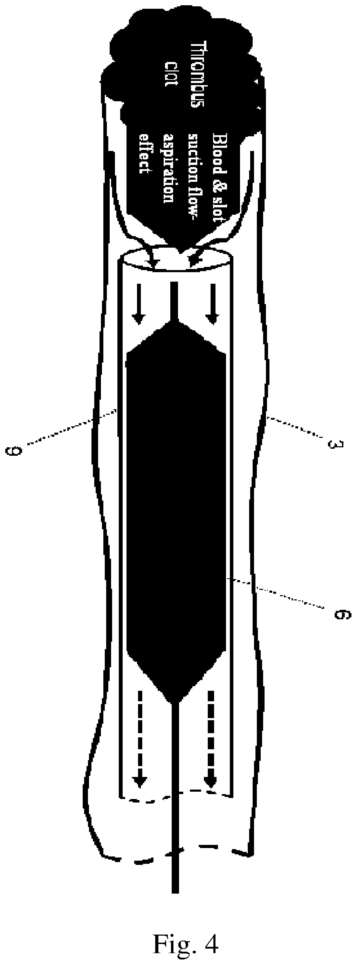

There is a secondary functional feature of the Delivery assist wire-catheter assembly - pull back aspiration effect (Fig. 4).

Once initial function of the Delivery assist wire-catheter assembly is achieved (drive and delivery of aspiration catheter to the thrombus area) then a secondary function of mentioned assembly is engaged.

When the Delivery assist wire (1) by continuous movement is being pulled back out of aspiration catheter tube (9), while aspi catheter is on still hold, the negative pressure as aspiration effect is immediately created without using any additional devices (e.g. syringes or aspiration pump machines).

The aspiration effect in the aspiration catheter starts sucking in blood and thus the nearby thrombus should be sucked into the (or should attach to the distal tip of the aspi catheter) aspi catheter and therefore it should enable to remove the thrombus clot out of the vessel which is the major target of thrombectomy procedure.

The Delivery assist guide wire-catheter assembly of this invention provides easy to use and easy to manoeuvre the delivery assist guide wire-catheter assembly to reach thrombus area and to remove thrombus from the vessels of human or animal body.

The above description is only preferred embodiments of the present disclosure and are not to be used to limit the present disclosure. It will be apparent to those skilled in the art that various modifications and variations can be made in the present disclosure, wherein the invention is defined by the appended claims.

## Claims

1. A Delivery assist wire-catheter assembly, which comprises:
an aspiration catheter having longitudinal tube (2), and
a Delivery assist wire (1) which is configured for insertion into the longitudinal tube (2) of the aspiration catheter,
wherein the Delivery assist wire (1) has a distal tip part (4), a distal working part (6) and a base part (8) and wherein the base part (8) of the Delivery assist wire (1) has a smaller diameter relative to the diameter of the distal working part (6) of the Delivery assist wire (1),
wherein the base part (8) of the Delivery assist wire (1) has a diameter maximal 1 mm, preferably 0.89 mm and the distal working part (6) has a diameter in an interval between 0.9 mm and 1.88 mm, preferably 1.27 mm; and
wherein the distal working part (6) of the Delivery assist wire (1) has an outer diameter from 60 to 99 percent of an average inner diameter of an aspiration catheter lumen at the distal end of the aspiration catheter.

2. The Delivery assist wire-catheter assembly according to claim 1, wherein the distal tip part (4) of the Delivery assist wire (1) has a diameter maximal 1 mm, preferably 0.89 mm.

3. The Delivery assist wire-catheter assembly according to claim 1 or 2, wherein the Delivery assist wire (1) has two additional parts (5, 7) which consist of a distal inclining conical part and a proximal declining conical or a negative conical or perpendicular shape part.

4. The Delivery assist wire-catheter assembly according to any of claims 1 - 3, wherein a structure of a guide wire is selected from core wire and a spring wire mounted on a top of the core or braided of multiple wires forming a single body of the Delivery assist wire (1).

5. The Delivery assist wire-catheter assembly according to claim 4, wherein the spring wire or braided wire has a hydrophilic coating.

6. The Delivery assist wire-catheter assembly according to any of claims 1 - 5, wherein the Delivery assist wire (1) is configured for insertion into the aspiration catheter.

7. The Delivery assist wire-catheter assembly according to any of claims 1 - 6, wherein the guide wire comprises Nitinol or stainless steel.

8. The Delivery assist wire-catheter assembly according to any of claims 1 - 7, wherein the Delivery assist wire (1) is configured for insertion into the aspiration catheter and wherein the aspiration catheter is configured to reach a thrombus following to the path of Delivery assist wire (1) (step-by-step).

9. The Delivery assist wire-catheter according to any of claims 1 - 7, wherein the Delivery assist wire (1) is configured for insertion into the aspiration catheter and wherein the aspiration catheter and the guide wire are configured for simultaneous reaching of the thrombus (uni-body).

10. The Delivery assist wire-catheter assembly according to any of preceding claims, wherein the Delivery assist wire (1) is configured to work as a support body for a proximal end and a middle end of the aspiration catheter to keep on pushing the aspiration catheter forward.

11. The Delivery assist wire-catheter assembly according to any of preceding claims, wherein the Delivery assist wire (1), by continuous movement, is configured to be pulled back out of the aspiration catheter, while the aspiration catheter is on still hold, creating negative pressure.

12. The Delivery assist wire-catheter assembly according to any of preceding claims, wherein the Delivery assist wire-catheter assembly is configured to remove a thrombus clot out of a vessel without any additional devices.

13. The Delivery assist wire-catheter assembly according to any of preceding claims, which is configured for use in thrombectomy as an aspiration neuro catheter.

14. The Delivery assist wire-catheter assembly according to any of preceding claims, which is configured for use to steer, drive and advance forward the aspiration catheter to an intracranial vessel area from a carotid up to M1-M3 vessel segments.

## Patentansprüche

1. Einführhilfe-Draht-Katheter-Anordnung, die umfasst:
einen Absaugkatheter mit einem Längsrohr (2) und
einen Einführhilfe-Draht (1), der zum Einführen in das Längsrohr (2) des Absaugkatheters konfiguriert ist,
wobei der Einführhilfe-Draht (1) einen distalen Spitzenteil (4), einen distalen Arbeitsteil (6) und einen Basisteil (8) aufweist und wobei der Basisteil (8) des Einführhilfe-Drahtes (1) einen kleineren Durchmesser relativ zu dem Durchmesser des distalen Arbeitsteils (6) des Einführhilfe-Drahtes (1) aufweist,
wobei der Basisteil (8) des Einführhilfe-Drahtes (1) einen Durchmesser von maximal 1 mm, vorzugsweise 0,89 mm, hat und der distale Arbeitsteil (6) einen Durchmesser in einem Intervall zwischen 0,9 mm und 1,88 mm, vorzugsweise 1,27 mm, hat; und
wobei der distale Arbeitsteil (6) des Einführhilfe-Drahtes (1) einen Außendurchmesser von 60 bis 99 Prozent eines durchschnittlichen Innendurchmessers eines Absaugkatheterlumens am distalen Ende des Absaugkatheters aufweist.

2. Einführhilfe-Draht-Katheter-Anordnung nach Anspruch 1, wobei der distale Spitzenteil (4) des Einführhilfe-Drahtes (1) einen Durchmesser von maximal 1 mm, vorzugsweise 0,89 mm, aufweist.

3. Einführhilfe-Draht-Katheter-Anordnung nach Anspruch 1 oder 2, wobei der Einführhilfe-Draht (1) zwei zusätzliche Teile (5, 7) aufweist, die aus einem distalen, geneigten, konischen Teil und einem proximalen, abfallenden, konischen oder negativ konischen oder senkrecht geformten Teil bestehen.

4. Einführhilfe-Draht-Katheter-Anordnung nach einem der Ansprüche 1 bis 3, wobei eine Struktur eines Führungsdrahtes ausgewählt ist aus einem Kerndraht und einem Federdraht, der oben auf dem Kern angebracht ist oder aus mehreren Drähten geflochten ist, die einen einzigen Körper des Einführhilfe-Drahtes (1) bilden.

5. Einführhilfe-Draht-Katheter-Anordnung nach Anspruch 4, wobei der Federdraht oder der geflochtene Draht eine hydrophile Beschichtung aufweist.

6. Einführhilfe-Draht-Katheter-Anordnung nach einem der Ansprüche 1 bis 5, wobei der Einführhilfe-Draht (1) zum Einführen in den Absaugkatheter konfiguriert ist.

7. Einführhilfe-Draht-Katheter-Anordnung nach einem der Ansprüche 1 bis 6, wobei der Führungsdraht Nitinol oder rostfreien Stahl umfasst.

8. Einführhilfe-Draht-Katheter-Anordnung nach einem der Ansprüche 1 bis 7, wobei der Einführhilfe-Draht (1) zum Einführen in den Absaugkatheter konfiguriert ist und wobei der Absaugkatheter so konfiguriert ist, dass er einen Thrombus erreicht, der dem Weg des Einführhilfe-Drahtes (1) (Schritt-für-Schritt) folgt.

9. Einführhilfe-Draht-Katheter nach einem der Ansprüche 1 bis 7, wobei der Einführhilfe-Draht (1) zum Einführen in den Absaugkatheter konfiguriert ist und wobei der Absaugkatheter und der Führungsdraht zum gleichzeitigen Erreichen des Thrombus (uni-body) konfiguriert sind.

10. Einführhilfe-Draht-Katheter-Anordnung nach einem der vorhergehenden Ansprüche, wobei der Einführhilfe-Draht (1) so konfiguriert ist, dass er als Stützkörper für ein proximales Ende und ein mittleres Ende des Absaugkatheters wirkt, um den Absaugkatheter weiter vorwärts zu schieben.

11. Einführhilfe-Draht-Katheter-Anordnung nach einem der vorhergehenden Ansprüche, wobei der Einführhilfe-Draht (1) so konfiguriert ist, dass er durch eine kontinuierliche Bewegung aus dem Absaugkatheter herausgezogen wird, während der Absaugkatheter in Ruhe gehalten wird, wodurch ein Unterdruck erzeugt wird.

12. Einführhilfe-Draht-Katheter-Anordnung nach einem der vorhergehenden Ansprüche, wobei die Einführhilfe-Draht-Katheter-Anordnung so konfiguriert ist, dass sie ein Thrombusgerinnsel aus einem Gefäß ohne zusätzliche Vorrichtungen entfernt.

13. Einführhilfe-Draht-Katheter-Anordnung nach einem der vorhergehenden Ansprüche, die für eine Verwendung in der Thrombektomie als Absaugneurokatheter konfiguriert ist.

14. Einführhilfe-Draht-Katheter-Anordnung nach einem der vorhergehenden Ansprüche, die so konfiguriert ist, dass sie zum Steuern, Antreiben und Vorschieben des Absaugkatheters zu einem intrakraniellen Gefäßbereich von einer Karotis bis zu M1-M3-Gefäßsegmenten verwendet werden kann.

## Revendications

1. Ensemble fil-cathéter d'aide à la mise en place, qui comprend :
un cathéter d'aspiration comportant un tube longitudinal (2), et
un fil d'aide à la mise en place (1) qui est conçu pour une insertion dans le tube longitudinal (2) du cathéter d'aspiration,
dans lequel le fil d'aide à la mise en place (1) comporte une partie pointe distale (4), une partie de travail distale (6) et une partie base (8) et dans lequel la partie base (8) du fil d'aide à la mise en place (1) a un plus petite diamètre par rapport au diamètre de la partie de travail distale (6) du fil d'aide à la mise en place (1),
dans lequel la partie base (8) du fil d'aide à la mise en place (1) a un diamètre maximal de 1 mm, de préférence de 0,89 mm et la partie de travail distale (6) a un diamètre dans un intervalle compris entre 0,9 mm et 1,88 mm, de préférence de 1,27 mm ; et
dans lequel la partie de travail distale (6) du fil d'aide à la mise en place (1) a un diamètre extérieur de 60 à 99 pour cent d'un diamètre intérieur moyen d'une lumière de cathéter d'aspiration au niveau de l'extrémité distale du cathéter d'aspiration.

2. Ensemble fil-cathéter d'aide à la mise en place selon la revendication 1, dans lequel la partie pointe distale (4) du fil d'aide à la mise en place (1) a un diamètre maximal de 1 mm, de préférence de 0,89 mm.

3. Ensemble fil-cathéter d'aide à la mise en place selon la revendication 1 ou 2, dans lequel le fil d'aide à la mise en place (1) comporte deux parties supplémentaires (5, 7) qui sont constituées d'une partie conique inclinée distale et d'une partie conique déclive proximale ou d'une partie conique négative ou d'une partie de forme perpendiculaire.

4. Ensemble fil-cathéter d'aide à la mise en place selon l'une quelconque des revendications 1 à 3, dans lequel une structure d'un fil-guide est choisie parmi un fil central et un fil à ressort monté sur le dessus du noyau ou tressé de plusieurs fils formant un corps unique du fil d'aide à la mise en place (1).

5. Ensemble fil-cathéter d'aide à la mise en place selon la revendication 4, dans lequel le fil à ressort ou le fil tressé a un revêtement hydrophile.

6. Ensemble fil-cathéter d'aide à la mise en place selon l'une quelconque des revendications 1 à 5, dans lequel le fil d'aide à la mise en place (1) est conçu pour une insertion dans le cathéter d'aspiration.

7. Ensemble fil-cathéter d'aide à la mise en place selon l'une quelconque des revendications 1 à 6, dans lequel le fil-guide comprend du Nitinol ou de l'acier inoxydable.

8. Ensemble fil-cathéter d'aide à la mise en place selon l'une quelconque des revendications 1 à 7, dans lequel le fil d'aide à la mise en place (1) est conçu pour une insertion dans le cathéter d'aspiration et dans lequel le cathéter d'aspiration est conçu pour atteindre un thrombus en suivant le trajet du fil d'aide à la mise en place (1) (étape par étape).

9. Fil-cathéter d'aide à la mise en place selon l'une quelconque des revendications 1 à 7, dans lequel le fil d'aide à la mise en place (1) est conçu pour une insertion dans le cathéter d'aspiration et dans lequel le cathéter d'aspiration et le fil-guide sont conçus pour atteindre simultanément le thrombus (uni-corps).

10. Ensemble fil-cathéter d'aide à la mise en place selon l'une quelconque des revendications précédentes, dans lequel le fil d'aide à la mise en place (1) est conçu pour fonctionner en tant que corps de support pour une extrémité proximale et une extrémité médiane du cathéter d'aspiration pour continuer à pousser le cathéter d'aspiration vers l'avant.

11. Ensemble fil-cathéter d'aide à la mise en place selon l'une quelconque des revendications précédentes, dans lequel le fil d'aide à la mise en place (1), par un mouvement continu, est conçu pour être retiré du cathéter d'aspiration, tandis que le cathéter d'aspiration est toujours en place, créant une pression négative.

12. Ensemble fil-cathéter d'aide à la mise en place selon l'une quelconque des revendications précédentes, dans lequel l'ensemble fil-cathéter d'aide à la mise en place est conçu pour retirer un caillot de thrombus d'un vaisseau sans aucun dispositif supplémentaire.

13. Ensemble fil-cathéter d'aide à la mise en place selon l'une quelconque des revendications précédentes, qui est conçu pour une utilisation en thrombectomie en tant que neurocathéter d'aspiration.

14. Ensemble fil-cathéter d'aide à la pose selon l'une quelconque des revendications précédentes, qui est conçu pour une utilisation pour diriger, entraîner et faire avancer le cathéter d'aspiration vers une zone de vaisseau intracrânien depuis une carotide jusqu'à des segments de vaisseau M1-M3.
